# EUROPEAN PATENT APPLICATION

(11) **EP 2 545 909 A1**
(43) Date of publication of application: **16.01.2013**
(21) Application number: 11173618.7
(22) Date of filing: 12.07.2011
(51) Int. Cl.: A61K 31/00, A61K 31/198, A61K 31/7004, A61K 31/7016, A61P 35/00

(54) **Combination of D-Amino Acids and Lipoteichoic Acid**

(71) Applicant: Lunamed AG, 7000 Chur (CH)
(72) Inventor: TRUOG, Peter, 7000 Chur (CH)
(74) Representative: Peters, Hajo

(57) **Abstract**

The present invention refers to combining teichoic acid, especially lipoteichoic acid or its salts and at least one D-amino acid, e.g. in a pharmaceutical composition, pharmaceutical formulations comprising such a composition as well as their use in cancer therapy and in other pharmaceutical applications.

## Description

### Field of the invention

The present invention refers to combining teichoic acid, especially lipoteichoic acid or its salts and at least one D-amino acid, e.g. in a pharmaceutical composition, pharmaceutical formulations comprising such a composition as well as their use in cancer therapy and in other pharmaceutical applications.

### Background of the invention

Cancer is a one of the most dreaded and feared diseases capturing the intense interest of nearly all levels of society but also causing enormous cost to health care. It is one of the most common causes of death worldwide. The efforts to fight or to contain cancer are considerably and thus treatment of cancer is one of the most important targets of modern medicine. Even though a lot of medical treatments have been presented so far and over the last decades, with not a few of them with extreme grave consequences to the patient, there is still a big need for a treatment going beyond the usual treatment known today ameliorating it effect and minimizing the side effects. Thus, the governing and overall goal of this invention was thus improvement of cancer treatment in general.

Lipoteichoic acid (LTA) is a gram positive pathogen cell wall motif that allows the pathogen to be immunologically recognised by binding to Toll-like receptors on the cell surface. LTAs have been reported as having anti tumor activity (EP 135820; USP 4,678,773; A. Yamamoto et. al. 1985, Br. J. Cancer, 51, 739 - 742; and H. Usami et. al., Br. J. Cancer, 1988, 57, 70 - 73) and especially also WO 96/023896. Even though such treatments had shown good effects, there had also been cases and reports in literature in which such a use of lipoteichoic acid or of its sodium salt were less effective or even showed no effect in the treatment of cancer.

Accordingly, it was the goal of this invention to identify ways of using lipoteichoic acid or its salts in an improved way in the treatment of various diseases, especially cancer or cancer subtypes. Such improvements would include improving the clinical results seen in patients after treatment with lipoteichoic acid or its salts or hydrates such as prolongation of survival time, reduction or even removal of cancer cells, allowing patients to be effectively treated that were not or only to a limited degree treatable before, reducing side-effects seen in patients suffering from cancer or increasing or decreasing levels of biomarkers used as parameters to indicate success in such a cancer treatment with these improvements being shown either in one or more of the above listed aspects. Such an improvement could further also include a reduction in dose of each treatment with lipoteichoic acid or its salts or hydrates, reducing the number of application per day of lipoteichoic acid or its salts or hydrates or even reducing any side effect possibly occurring during such a treatment (even though treatment with lipoteichoic acid or its salts is known to have close to no side effects).

It has now been found that combining teichoic acids (or lipoteichoic acid) as free acid or any of its salts or hydrates with at least one D-amino acids (especially D-alanine) did show clear advantages in the treatment of various diseases such as cancer.

Accordingly, the invention is drawn to combining lipoteichoic acid or any of its salts or hydrates with at least one D-amino acids or any of its salts. Combining lipoteichoic acid or any of its salts or hydrates with at least one D-amino acids or any of its salts according to the invention is drawn to different embodiments and also aspects.

Thus, one aspect of the invention is drawn to combining the lipoteichoic acid in any variant as free acid or as any pharmaceutically acceptable salt or hydrate thereof with at least one D-amino acids as free acid or as any pharmaceutically acceptable salt or hydrate thereof by applying the lipoteichoic acid in any of its variants or any of its salts or hydrates and the D-amino acids or any of its salts e.g. in the treatment of a patient in need thereof. Thus, this aspect of the invention would encompass a method of treatment of cancer by providing to a patient in need thereof a sufficient amount of teichoic or lipoteichoic acid (especially LTA-T) as free acid or as any pharmaceutically acceptable salt or hydrate thereof and a sufficient amount of the D-amino acids as free acid or as any pharmaceutically acceptable salt or hydrate thereof. Preferably this provision of the two amounts of lipoteichoic acid or any of its salts or hydrates on one hand and of the D-amino acids or any of its salts on the other hand is done simultaneously or in a short time span, like one provision immediately following the other. Preferably this provision of the two amounts of lipoteichoic acid or any of its salts or hydrates on one hand and of the D-amino acids or any of its salts on the other hand is done orally for the D-amino acid (D-Alanine) and by injection for the teichoic acid or lipoteichoic acid (especially LTA-T). Preferably the D-amino acid (or salt thereof) is D-alanine. Preferably the injectable dosage form contains between 0.1 and 10 µmol, preferably 0.5 to 2.0 µmol, most preferably 1.0 µmol teichoic acid/ml. The amount of the D-amino acid (preferably of the D-alanine) used within this treatment is 10 to 500 mg, preferably 40 to 250 mg or 50 mg of D-amino acid or any of its salts (like especially D-alanine) preferably in one dose with this dose being preferably given (preferably orally) once or twice daily, most preferably twice daily. Preferably the teichoic acid in the dosage form for injection is preferably dissolved in a physiologically acceptable aqueous medium, most preferably in physiological saline (NaCl 0.9%). The injectable dosage forms may be used for single or repeated (from 1 to 10 times per day) injections including subcutaneous, intraperitoneal or intrapleural injection etc.

Another aspect of the invention is drawn to combining the teichoic acid or lipoteichoic acid or any of its salts or hydrates with at least one D-amino acids or any of its salts by applying the teichoic acid or lipoteichoic acid (especially LTA-T) or any of its salts or hydrates and the D-amino acids or any of its salts in a physically separate form e.g. in the treatment of a patient in need thereof. Thus, this aspect of the invention would encompass a method of treatment of cancer (or any of the other indications listed in later paragraphs) by providing to a patient in need thereof a sufficient amount of lipoteichoic acid as free acid or as any pharmaceutically acceptable salt or hydrate thereof and - in physically separate form - a sufficient amount of the D-amino acids as free acid or as any pharmaceutical acceptable salt or hydrate thereof. Preferably this provision of the two amounts of lipoteichoic acid or any of its salts or hydrates on one hand and of the D-amino acids or any of its salts on the other hand is done simultaneously or in a short time span, like one provision immediately following the other. Preferably this provision of the two amounts of lipoteichoic acid or any of its salts or hydrates on one hand and of the D-amino acids or any of its salts on the other hand is done orally for the D-amino acid (D-Alanine) and by injection for the teichoic acid or lipoteichoic acid (especially LTA-T). Preferably the D-amino acid (or salt thereof) is D-alanine. Preferably the injectable dosage form contains between 0.1 and 10 µmol, preferably 0.5 to 2.0 µmol, most preferably 1.0 µmol teichoic acid/mi. The amount of the D-amino acid (preferably of the D-alanine) used within this treatment is 10 to 500 mg, preferably 40 to 250 mg or 50 mg of D-amino acid or any of its salts (like especially D-alanine) preferably in one dose with this dose being preferably given (preferably orally) once or twice daily, most preferably twice daily. Preferably the teichoic acid in this dosage form for injection is preferably dissolved in a physiologically acceptable aqueous medium, most preferably in physiological saline (NaCi 0.9%). The injectable dosage forms may be used for single or repeated (from 1 to 10 times per day) injections including subcutaneous, intraperitoneal or intrapleural injection.

A central aspect of this invention is drawn to a pharmaceutical composition comprising at least one teichoic acid (especially a lipoteichoic acid) as free acid or as any pharmaceutically acceptable salt or hydrate thereof and at least one D-amino acid as free acid or as any pharmaceutically acceptable salt or hydrate thereof. A preferred embodiment of this central aspect of this invention is drawn to a pharmaceutical composition comprising at least one lipoteichoic acid as free acid or as any pharmaceutically acceptable salt or hydrate thereof and at least one D-amino acid as free acid or as any pharmaceutically acceptable salt or hydrate thereof. Accordingly in a preferred embodiment of the pharmaceutical composition according to the central aspect of the invention the teichoic acid is a lipoteichoic acid.

"teichoic acids" as used herein is an inclusive term including the a) lipoteichoic acids (LTA) and b) the wall teichoic acids (WTA) form a major part of the cell wall of gram-positive bacteria.

"Lipoteichoic acid" - also abbreviated as "LTA" used herein is an inclusive term including the a) lipoteichoic acids (lea) There are many lipoteichoic acids known which form a major part of the cell wall of gram-positive bacteria. The known lipoteichoic acids include synthetic, semisynthetic and naturally occurring lipoteichioic acids. A very broad introduction on the synthesis of as well as on synthetic lipoteichoic acids can be found in Pedersen, Christian Marcus; Schmidt, Richard R. (Edited by Moran, Anthony P), Microbial Glycobiology (2009). 455-476 with all lipoteichoic acids described therein herewith included by reference in this application as examples of the lipoteichoic acids to be used. A further overview of the lipoteichoic acids - divided in Types 1 to IV - can be found in Greenberg et al., Infect Immun. 1996 Aug;64(8):3318-25. All lipoteichoic acids Type 1, Type 11, Type III and Type IV as well as all those described therein (like e.g. in tables 1 or 2) herewith included by reference in this application as examples of the lipoteichoic acids to be used.

LTAs were isolated from e. g. Lactobacillus heiveticus (NCIB 8025), Lactobacillus fermenti (NCTC 6991), Streptococcus faecalis, 39, Streptococcus lactis (ATCC 9936), Streptococcus mutans, AHT (A. J. Wicken et al, 1975), and Streptococcus pyogenes SV strain (ATCC 21059) (EP 135 820, USP 4,678,773, H. Usami et. al. 1985).

In one preferred embodiment of the pharmaceutical composition according to (the central aspect of) the invention the teichoic acid is a (general) lipoteichoic acid. The (general) lipoteichoic acids as defined in this application are comprising:
a) a linear, hydrophilic 1,3-connected glycero-phophate chain - optionally substituted with D-alanine or other molecules -,
b) the gylcerophosophate chain being covalently bound through a phosphodiester to
c) a glycolipid, preferably a glyceroglycolipid,

Lipoteichoic acids falling under this definition according to this invention are called "(general) lipoteichoic acids" especially hereinafter.

The glycolipid acts as an anchor in the cell membrane. The glycolipids are by definition of the invention lipids of the membrane in which one or more mono- or oligosaccharids are bound via a glycosidic bond to a lipid molecule. The lipid molecule is consisting of fatty acids being bound by an ester bond to a glycerol or by an amid bond to a sphingosine.

In one preferred embodiment of the invention relating to a (general) lipoteichoic acid the glycolipid is a glyceroglycolipid in which one or more mono- or oligosaccharids are bound via a glycosidic bond to a lipid molecule consisting of fatty acids being bound by an ester bond to the glycerol. Preferably the glyceroglycolipid comprises a diacylglycerol with two fatty acid chains of 10 to 20 carbon atoms each being bound by an ester bond to the glycerol; and/or preferably the fatty acid chains are linear or branched and non-substituted and/or have a length of 10 to 20 carbon atoms each, most preferably 12, 14, 16 or 18 carbon atoms.

Most (general) lipoteichoic acids would be covered by the general formula I and its substituents as described and defined below and some preferred embodiments are described below, but some are unusual. These unusual include the lipoteichoic acid from *Streptococcus pneumoniae* (see below) whose synthesis was recently described by Pedersen et al., in Angew. Chem. (2010), 122, 1 - 7. 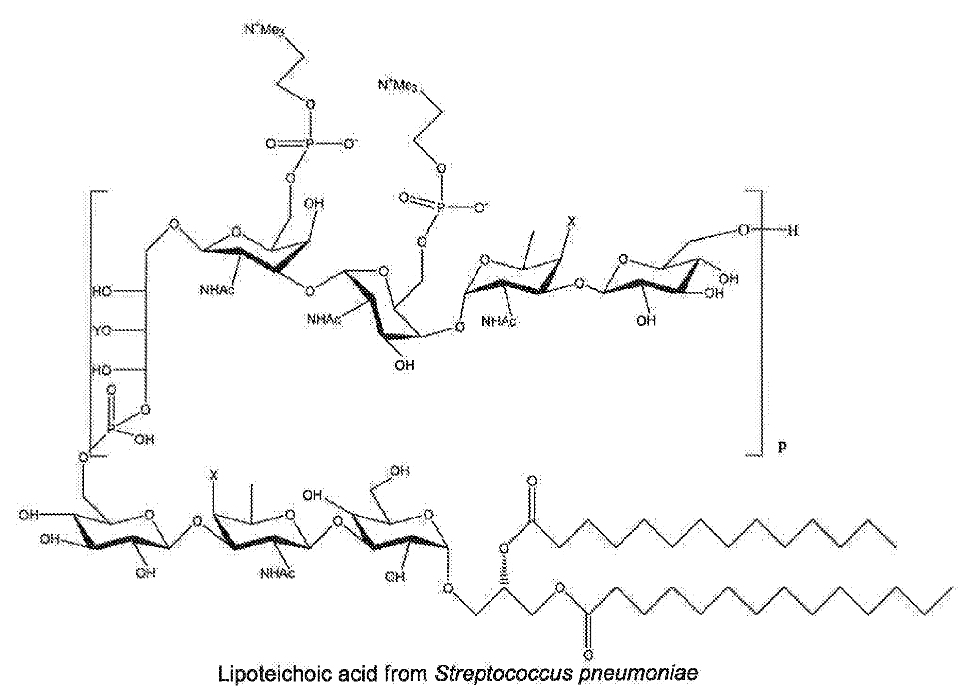 wherein
in one case X is NH₃⁺, or NHAc; Y is H, D-Ala, or a-GalNAc; and p is up to 8;
in the other case X is NH₃⁺; Y is H; and p is 1.

In one preferred embodiment of the pharmaceutical composition according to (the central aspect of) the invention the teichoic acid is a lipoteichoic acid according to general formula I 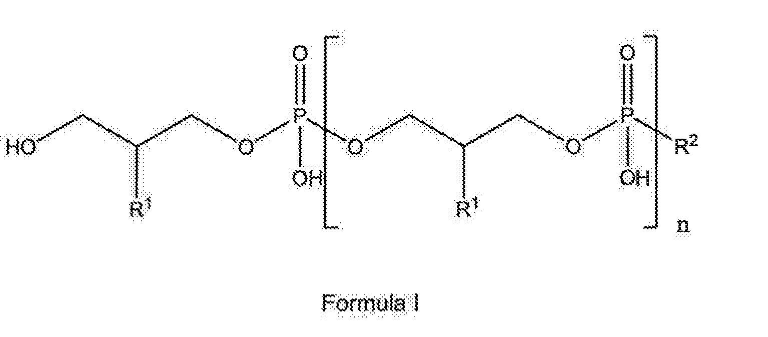 wherein
the mean value of n is 8 to 40,
R¹ is either OH or O-D-alanyl and
R² is
either 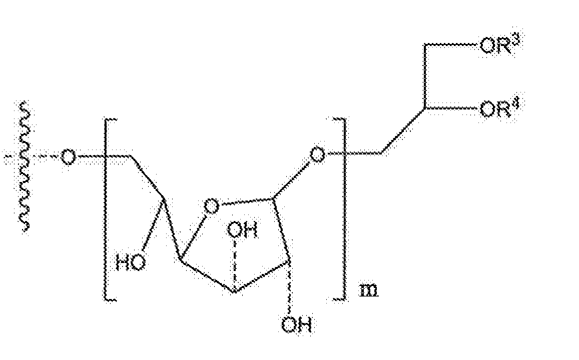 or its tautomeric equivalent 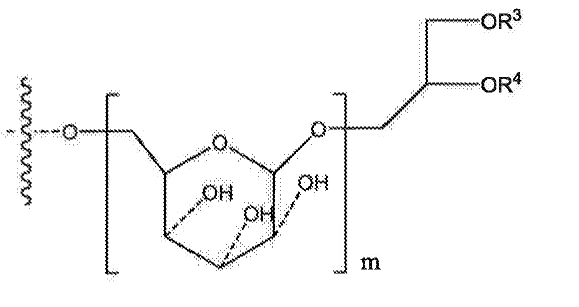 with R³ and R4 independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures.

In one even more preferred embodiment of the pharmaceutical composition according to (the central aspect of) the invention the teichoic acid is a lipoteichoic acid according to general formula I 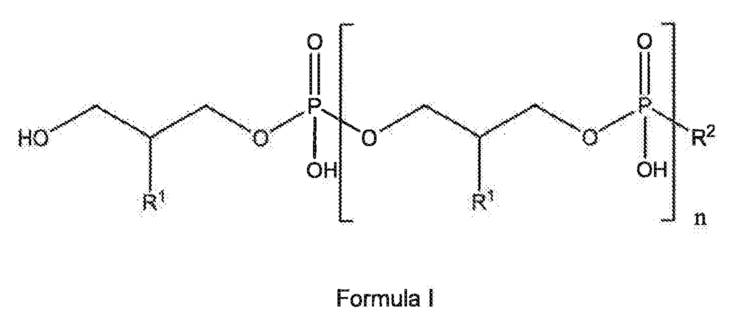 wherein
the mean value of n is 9,
R¹ is either OH or O-D-Alanyl and
R² is 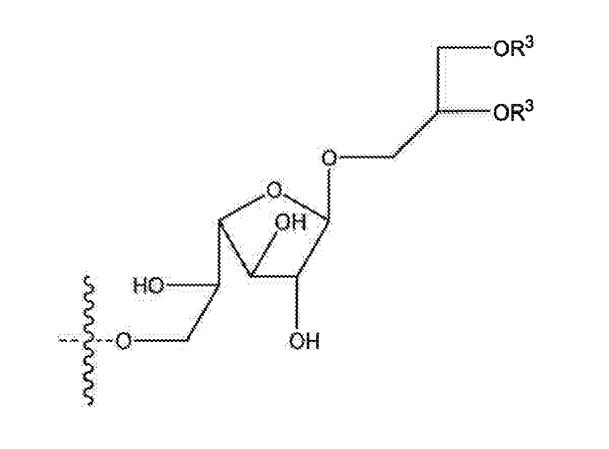 with R³ being residues of saturated or unsaturated fatty acids with 12, 14, 16 for 18 carbon atoms,
with the compounds of formula I being present either in free form or as physiologically acceptable salt; in form of any of their structural isomers including mixtures thereof, preferably in pure enantiomeric or diastereomeric form or any mixture thereof including racemic mixtures. These compounds defined above as well as those described by general formula la, lb, lc or ld and their defined radicals below (as well as any lipoteichoic acid to be isolated from the Streptococcus sp. PT strain DSM 8747) are defined in this invention as "lipoteichoic acid of the invention".

In a further embodiment of the pharmaceutical composition according to (a central aspect of) the invention the teichoic acid is a purified lipoteichoic acid isolated or isolatable from the Streptococcus sp. PT strain DSM 8747, preferably containing a beta-galactofuranosyl(1-3)glycerol-di-ester moiety.

In one preferred embodiment of the pharmaceutical composition according to (the central aspect of) the invention the teichoic acid is a lipoteichoic acid according to any of general formulas la, lb, lc or ld 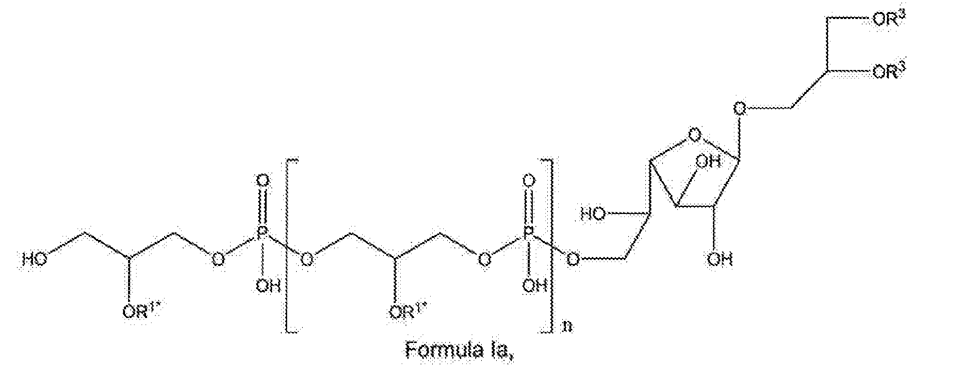 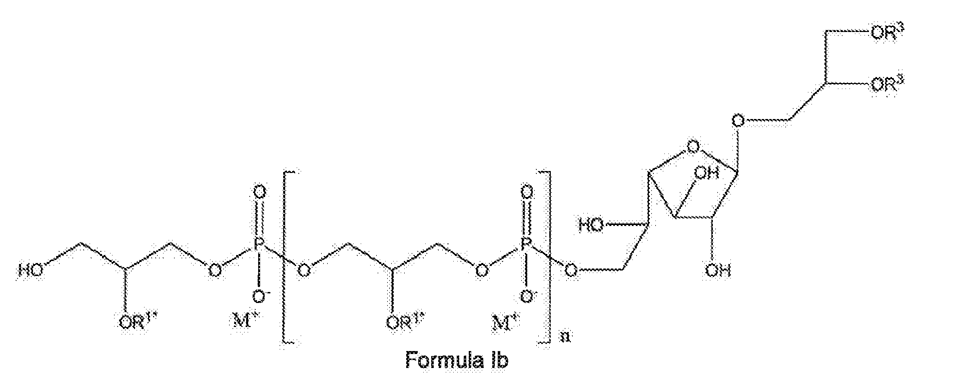 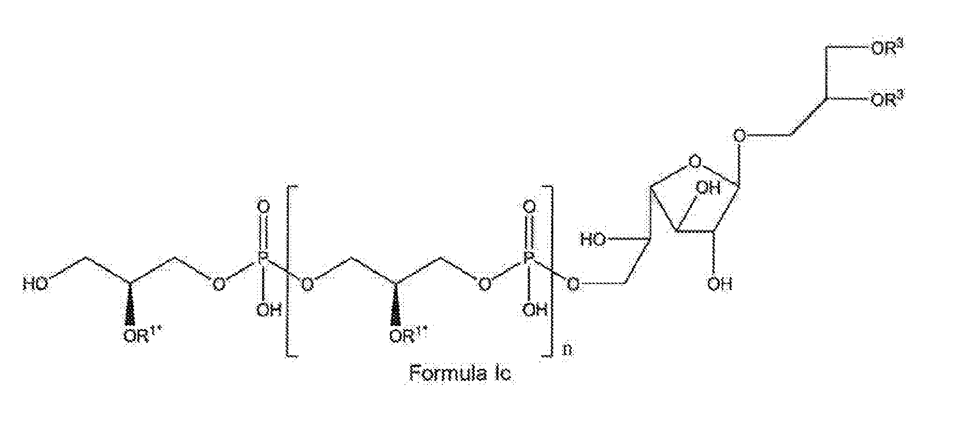 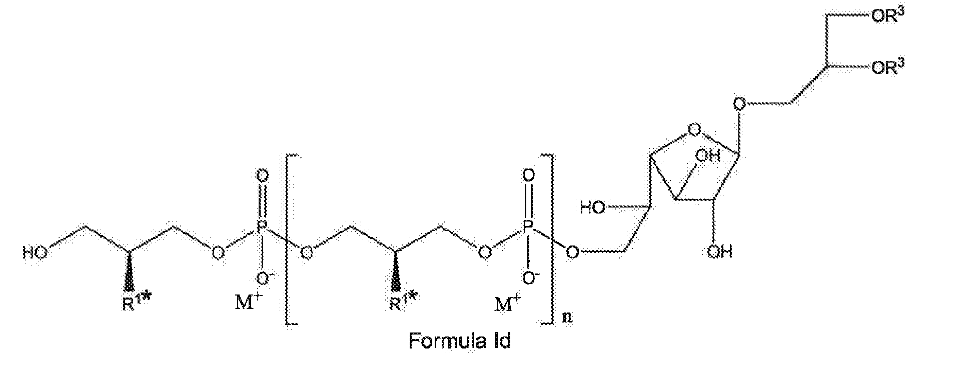 with
n having a mean value of 9, preferably being 6 to 12 while having a mean value of 9,
R¹' being either H or D-alanyl,
R³ being residues of saturated or unsaturated fatty acids with 12, 14, 16 or 18 carbon atoms, and
M⁺ being selected from positively charged ions, such as alkali metal ions or positively charged primary, secondary, tertiary or quaternary ammonium ions, preferably M⁺ being a sodium ion.

In one preferred embodiment of the pharmaceutical composition according to (the central aspect of) the invention the teichoic acid is a purified lipoteichoic acid isolated from bacteria of the genus streptococcus, preferably from *streptococcus sp*., most preferably from streptococcus sp. (DSM 8747). One example of such an isolated lipoteichoic acid is lipoteichoic acid T (LTA-T).

Lipoteichoic acids - especially the lipoteichoic acid T (LTA-T) - are described in the PCT-application WO 96/23896 together with its antitumor cholesterol-lowering activity and ways of isolating the LTA-T. The whole disclosure of WO06/23896 is included in this application by way of reference.

LTA-T is a gram positive cell wall motif that allows it to be immunologically recognised by binding to Toll-like receptors on the cell surface. LTA-T is described in US 6,114,161 for use as an antitumour preparation. All aspects of LTA-T from US 6,114,161 are incorporated herein by reference.

Preferably "lipoteichoic acid" is lipoteichoic acid T (LTA-T). Thus, in one very preferred embodiment of the pharmaceutical composition according to the invention the teichoic acid is lipoteichoic acid T (LTA-T).

Teichoic acid, lipoteichoic acid or especially lipoteichoic acid T (LTA-T) may form salts with other (positively) charged ions, in particular physiologically acceptable salts, such as alkali metal or alkaline earth metal salts, also heavy metal salts, such as zinc or iron salts, or primary, secondary, tertiary or quaternary ammonium salts (acid addition salts) . Such other salts are e. g. potassium, calcium, ammonium, mono-, di-, tri- or tetra-lower alkyl-, like methyl- or ethyl-, or methyl-ethyl, propyl- or butylammonium salts. A prefered salt of LTA-T is the sodium salt.

In another embodiment of the pharmaceutical composition according to the invention the teichoic acid is a lipoteichoic acid according to general formula I being a lipoteichoic acid of *staphylococcus* aureus (below): 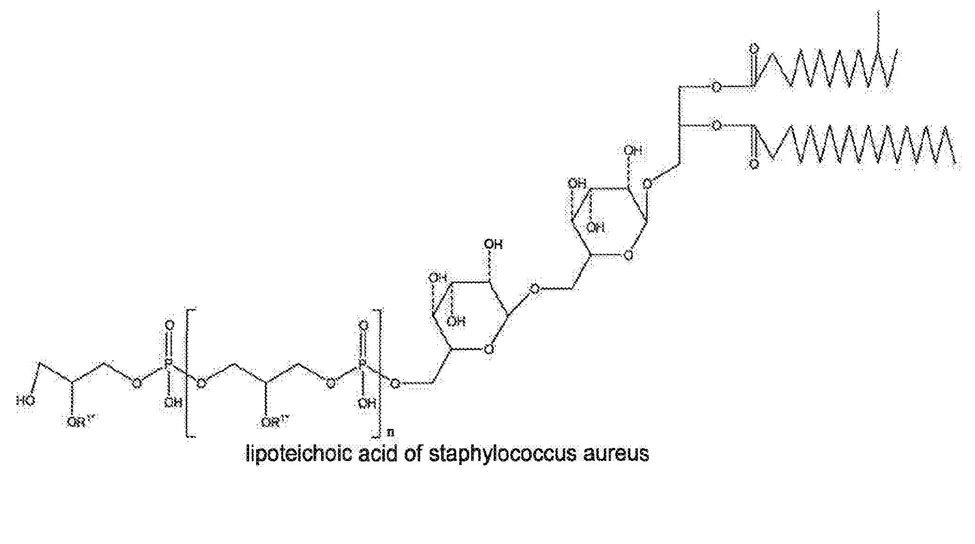 , wherein X is H or D-ala and the mean value of "n" is 16 to 40.

"Hydrate" is defined as a substance/joint mixture according to the invention containing water. Thus, it may be a crystalline salt with one or more water molecules in the crystalline structure or also amorphous containing water molecules.

"Solvate" is defined as a substance/joint mixture according to the invention containing a further solvent. One most prominent example of the solvents is an alcohol like ethanol or methanol thus leading to methanolates or ethanolates. Thus, it may be a crystalline salt with one or more alcohol molecules in the crystalline structure or also an amorphous form containing alcohol molecules.

In a further very preferred embodiment of the pharmaceutical composition according to (the central aspect of) the invention there is a fixed weight ratio between the teichoic acid or lipoteichoic acid as free acid or as any Pharmaceutical acceptable salt or hydrate thereof and the at least one D-amino acid as free acid or as any pharmaceutical acceptable salt or hydrate thereof regardless of which teichoic acid/lipoteichoic acid and which D-amino acid is finally selected in a given group of inventive pharmaceutical compositions I which one component (for example D-alanine for the D-amino acid) is fixed while the teichoic/lipoteichoic acid is varied.

In a further preferred embodiment of the pharmaceutical composition according to (the central aspect of) the invention the D-amino acid is selected from the group consisting of D-serine, D-alanine, D-lysine, D-arginine, D-glutamine D-asparagine or a pharmaceutically acceptable salt or hydrate thereof, preferably is selected from D-alanine.

In a further preferred embodiment of the pharmaceutical composition according to (the central aspect of) the invention the D-amino acid is D-alanine and the teichoic acid is lipoteichoic acid, preferably wherein the D-amino acid is D-alanine and the teichoic acid is lipoteichoic acid T.

In a preferred embodiment of the pharmaceutical composition according to (the central aspect of) the invention the content of lipoteichoic acid T will be between 0.1 to 10 µmol, 0.5 to 2.0 µmol or 1 µmol and the content of D-alanine will be 10 to 500 mg, preferably 40 to 250 mg or 50 mg.

Another aspect of the invention relates to a pharmaceutical formulation comprising the pharmaceutical composition according to according to (the central aspect of) the invention and optionally one or more pharmaceutically acceptable excipients.

In a preferred embodiment of the pharmaceutical formulation (or medicament) according to (the central aspect of) the invention the content of teichoic or lipoteichoic acid will be between 0.1 to 10 µmol, 0.5 to 2.0 µmol or 1 µmol and the content of D-amino acid will be 10 to 500 mg, preferably 40 to 250 mg or 50 mg, preferably the content of teichoic or lipoteichoic acid will be between 0.5 to 2.0 umol and the content of D-amino acid will be 40 to 250 mg, most preferably the content of teichoic or lipoteichoic acid will be ca. 1 µmol and the content of D-amino acid will be ca. 50 mg

In a very preferred embodiment of the pharmaceutical formulation (or medicament) according to (the central aspect of) the invention the content of lipoteichoic acid T will be between 0.I to 10 µmol, 0.5 to 2.0 µmol or 1 µmol and the content of D-alanine will be 10 to 500 mg, preferably 40 to 250 mg or 50 mg; preferably the content of lipoteichoic acid T will be ca. 1 µmol and the content of D-alanine will be ca. 50 mg.

The medicament or pharmaceutical formulation according to the present invention can be of any form suitable for the application to humans and/or animals, preferably humans including infants, children and adults and can be produced by standard procedures known to those skilled in the art. The medicament or pharmaceutical formulation can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament or pharmaceutical formulation of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical formulation may for example be injected intramuscularly, intraperitoneally, or intravenously.

Medicaments or pharmaceutical formulation according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, 1, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure. Medicaments or pharmaceutical formulation according to the present invention may also comprise an enteric coating, so that their dissolution is dependent on ph-value. Due to said coating the medicament may pass the stomach undissolved and the respective pharmaceutical composition according to the central aspect of the invention is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of the pharmaceutical composition according to the central aspect of the invention as defined herein and 40-99 % by weight of one or more auxiliary substances (additives/excipients).

The pharmaceutical combination of the central aspect of the present invention or medicament or pharmaceutical formulation of the invention may also be administered topically or via a suppository.

In a preferred embodiment of the pharmaceutical formulation according to the invention the pharmaceutical formulation is a prolonged release oral pharmaceutical dosage form.

In another preferred embodiment of the pharmaceutical formulation according to the invention the pharmaceutical formulation is a pharmaceutical dosage form prepared for injection.

In another preferred embodiment of the pharmaceutical formulation according to the invention in which the pharmaceutical formulation is a pharmaceutical dosage form prepared for injection, this dosage form contains between 0.1 and 10 µmol, preferably 0.5 to 2.0 µmol, most preferably 1.0 µmol teichoic acid/mi. The content of the D-amino acid (preferably of the D-alanine) is 10 to 500 mg, preferably 40 to 250 mg or 50 mg. The teichoic acid (and the D-amino acid) in this dosage form for injection is preferably dissolved in a physiologically acceptable aqueous medium, most preferably in physiological saline (NaCi 0.9%). Preferably the teichoic acid is lipoteichoic acid, most preferably is lipoteichoic acid T. These dosage forms [preferably being a solution of 0.1 to 10 µmol, 0.5 to 2.0 µmol (preferably 1.0 µmol) of the teichoic acid (lipoteichoic acid or LTA-T) and 10 to 500 mg, preferably 40 to 250 mg or 50 mg (preferably 50 mg) of D-amino acid (D-alanine) in physiologically acceptable aqueous medium like physiological saline] are used for single or repeated (from 1 to 10 times per day) injections. These injections include for example s.c. (subcutaneous), i.m. (intramuscular), i.v. (intravenous), i.a. (intra-arterial), intra-ocular, intra-pleural, intra-abdominal, or intra-thecal injection (depending on the clinical circumstances), with subcutaneous injection being slightly preferred.

The daily dosage for humans and animals may vary depending on factors that have their basis in the respective species or other factors, such as age, sex, weight or degree of illness and so forth. The daily dosage for humans preferably is in the range of 10 to 500 milligrams of D-amino acid and/or 0.1 to 50 µmol of teichoic acid (in the pharmaceutical combination according to the central aspect of the invention) to be administered during one or several intakes or injections per day.

In one further aspect of the invention the pharmaceutical composition according to the central aspect of the invention or the pharmaceutical formulation according to the invention is for use in the treatment of cancer, tumors, malignant cells, elevated cholesterol levels in blood, pleural effusion or pneumotnorax, preferably of cancer or pleural effusion; most preferably cancer or pleural effusion resulting from or combined with cancer.

"Pleural effusion" is defined herein as excess fluid that accumulates in the pleural cavity, the fluid-filled space that surrounds the lungs. Pleural effusions can be caused by a number of different diseases and conditions, such as cancer, heart failure, diseases causing low blood protein levels (for example liver cirrhosis and nephrotic syndrome), bacterial pneumonia, tuberculosis, pulmonary embolism, and diseases causing pleural inflammation (for example, systemic lupus erythematosus, rheumatoid arthritis and other autoimmune diseases), bleeding (often due to chest trauma), chylothorax, accidental infusion of fluids, oesophageal rupture or pancreatic disease, intra- abdominal abscess, rheumatoid arthritis, asbestos pleural effusion, Meig's syndrome and ovarian hyperstimulation syndrome.

"Pneumothorax" is defined herein as air in the pleural cavity. Pneumothorax can be caused by a number of different diseases and conditions, such as a penetrating chest wound, barotrauma to the lungs, chronic lung pathologies including emphysema, asthma, acute infections, acupuncture, chronic infections, such as tuberculosis, cancer and catamenial pneumothorax (due to endometriosis in the chest cavity), and can also arise without significant underlying lung disease in the form of a primary spontaneous pneumothorax

One other aspect of the invention is drawn to the use of the pharmaceutical composition according to the central aspect of the invention for the manufacture of a pharmaceutical formulations for the treatment cancer, tumors, malignant cells, elevated cholesterol levels in blood, pleural effusion or pneumothorax, preferably of cancer or pleural effusion; most preferably cancer or pleural effusion resulting from or combined with cancer

A further aspect of the invention would encompass a method of treatment of
cancer, tumors, malignant cells, elevated cholesterol levels in blood, pleural effusion or pneumothorax, preferably of cancer or pleural effusion; most preferably cancer or pleural effusion resulting from or combined with cancer
by providing to a patient in need thereof a sufficient amount of the pharmaceutical combination according to the central aspect of the invention. Preferably the provision of the amount of the pharmaceutical combination according to the central aspect of the invention is done in injectable form (a pharmaceutical formulation for parenteral, e.g. subcutaneous, intra-thecal, intra-abdominal or intrapieural injection). Preferably the injectable dosage form contains between 0.1 and 10 µmol, preferably 0.5 to 2.0 µmol, most preferably 1.0 µmol teichoic acid/mi. The amount of the D-amino acid (preferably of the D-alanine) in this dosage is 10 to 500 mg, preferably 40 to 250 mg or 50 mg. Preferably the teichoic acid (and the D-amino acid) in this dosage form for injection is preferably dissolved in a physiologically acceptable aqueous medium, most preferably in physiological saline (NaCi 0.9%). The injectable dosage forms may be used for single or repeated (from 1 to 10 times per day) injections including subcutaneous, intraabdominal, intraperitoneal or intrapleural injection.

One aspect of the invention would encompass a method of treatment or better of prophylaxis against (by lowering the risk of getting) cancer by providing to a patient in need thereof (at risk of suffering from cancer in the future or just having undergone a surgical cancer treatment or biopsys) a sufficient amount of the pharmaceutical combination according to the central aspect of the invention (or of both compounds in a combined treatment with teichoic acid and D-amino acid as described above) to treat non-encapsulated cancer cells. By this method non-encapsulated cancer cells are removed or transformed into non-malignant forms or forms undergoing apostosis if treated by the pharmaceutical combination according to the central aspect of the invention. "Non-encapsulated cancer cells are cancer/neoplastic cells not (yet) encapsulated by a hyaluronic acid capsule, like cells not accumulated or aggregated with other cancer cells or only accumulated or aggregated small groups of cancer cells not yet encapsulated up to microtumors.

One further aspect of the invention would encompass a method of treatment of cancer (or the surrounding tissue) in an inflammatory state by providing to a patient in need thereof a sufficient amount of the pharmaceutical combination according to the central aspect of the invention (or of both compounds in a combined treatment with teichoic acid and D-amino acid as described above). This would include a treatment after induction of inflammation in the cancer (or in the surrounding tissue). This would also include a treatment in which the non-encapsulated cancer (or the surrounding tissue) is pro-inflammatorically co-treated. The pro-inflammatory co-treatment might occur by (X-ray; iodizing) radiation or by application of at least one pro-inflammatory substance like pro-inflammatory interleukins. This inventive concept would also apply to a related aspect of the invention wherein the treatment is done by applying (e.g by (e.g. s.c.) injection of an injectable formulation) a pharmaceutical composition comprising at least one lipoteichoic acid (LTA-T) as free acid or as any pharmaceutically acceptable salt or hydrate thereof and by applying separately (e.g orally in an oral pharmaceutical formulation) before or after or simultaneously at least one D-amino acid (D-alanine) as free acid or as any pharmaceutically acceptable salt or hydrate thereof. This related aspect would naturally be also the subject of a method of treatment of cancer.

A last aspect of the invention would further encompass a method of treatment or better of prophylaxis against (by lowering the risk of getting) cancer by providing to a patient in need thereof (at risk of suffering from cancer in the future (due to non-encapsulated cancer cells) or accordingly just having undergone a surgical cancer treatment or biopsy) a sufficient amount of lipoteicoic acid, in which the patient is further co-treated (like local treatment e.g. at or around the area of surgery of biopsy) in a pro-inflammatory way and in which the treatment with lipoteichoic acid is done with a sufficient amount of the pharmaceutical combination according to the central aspect of the invention. Preferably this lipoteichoic acid is any of the lipoteichoic acids set out above and most preferably is LTA-T. The D-amino acid may be any D-amino acid but preferably is D-alanine. This pharmaceutical composition is preferably an injectable formulation comprising (e.g. 1 µmol) lipoteichoic acid (LTA-T) and (e.g. 50 mg) D-amino acid (D-alanine). The pro-inflammatory co-treatment might occur by (X-ray; iodizing) radiation or by application of at least one pro-inflammatory substance like pro-inflammatory interleukins.

The last aspect of the invention would also apply to a related aspect of the invention wherein the treatment is done by applying (e.g. by (e.g. s.c.) injection of an injectable formulation) a pharmaceutical composition comprising at least one lipoteichoic acid (LTA-T) as free acid or as any pharmaceutical acceptable salt or hydrate thereof and by applying separately (e.g. orally in an oral pharmaceutical formulation) before or after or simultaneously at least one D-amino acid (D-alanine) as free acid or as any pharmaceutically acceptable salt or hydrate thereof. This last aspect would naturally be also the subject of a method of treatment of cancer.

The present invention is illustrated below with the help of the following examples. These illustrations are given solely by way of example and do not limit the invention.

### EXAMPLES:

### Example 1: Pharmaceutical Formulation

The purified LTA-T in 0.05M sodiumacetate pH 4.7 (prepared as set out in WO 1996/023896) is dialysed extensively against physiological NaCl solution (0.9%) and the volume is adjusted to 1 µmol LTA-T (based on the phosphorus content) within 1 ml of physiological saline. 50 mg of D-alanine are added and disolved. After filtration of the solution through a filter membrane (Millipore 0.22um), 1 ml aliquots of the filtrate are placed in sterilized vials under sterile conditions. These vials contain 1 micromol/ml LEA-T phosphorus and 50 mg D-alanine and are used subcutaneously for therapeutical purposes.

## Claims

1. A pharmaceutical composition comprising at least one teichoic acid as free acid or as any pharmaceutically acceptable salt or hydrate thereof and at least one D-amino acid as free acid or as any pharmaceutically acceptable salt or hydrate thereof.

2. The pharmaceutical composition according to claim 1 wherein the teichoic acid is a lipoteichoic acid.

3. The pharmaceutical composition according to claim 2 wherein the lipoteichoic acid is comprising:
a) a linear, hydrophilic 1,3-connected glycero-phophate chain - optionally substituted with D-alanine or other molecules -,
b) the gylcerophosophate chain being covalently bound through a phosphodiester to
c) a glycolipid, preferably a glyceroglycolipid.

4. The pharmaceutical composition according to claim 3 wherein the glycolipid is a glyceroglycolipid in which one or more mono- or oligosaccharids are bound via a glycosidic bond to a lipid molecule consisting of fatty acids being bound by an ester bond to the glycerol.

5. The pharmaceutical composition according to claim 4 wherein the glyceroglycolipid comprises a diacylglycerol with two fatty acid chains of 10 to 20 carbon atoms each being bound by an ester bond to the glycerol,
and/or wherein the fatty acid chains being linear or branched and non-substituted and/or having a length of 10 to 20 carbon atoms each, most preferably of 12, 14, 16 or 18 carbon atoms.

6. The pharmaceutical composition according to claim 2 wherein the lipoteichoic acid is a structure according to general formula I 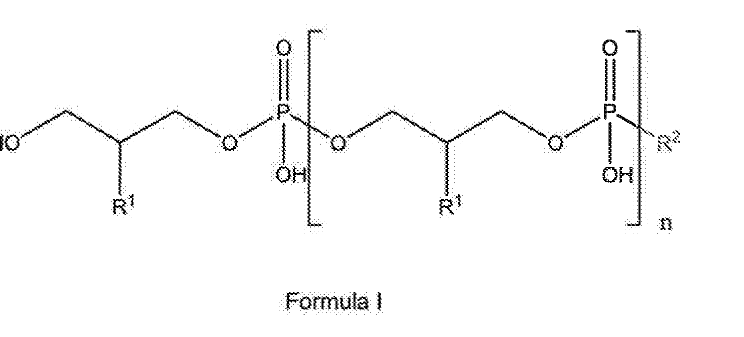 wherein
the mean value of n is 8 to 40,
R¹ is either OH or O-D-alanyl and
R² is
either 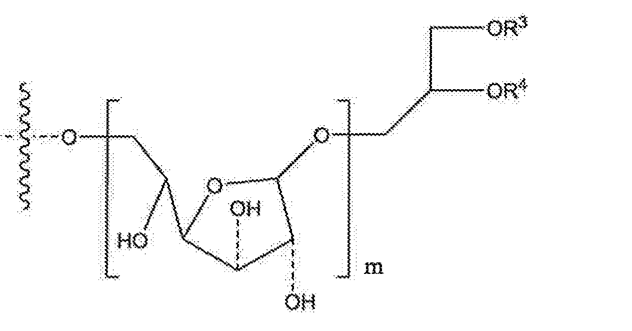 or its tautomeric equivalent 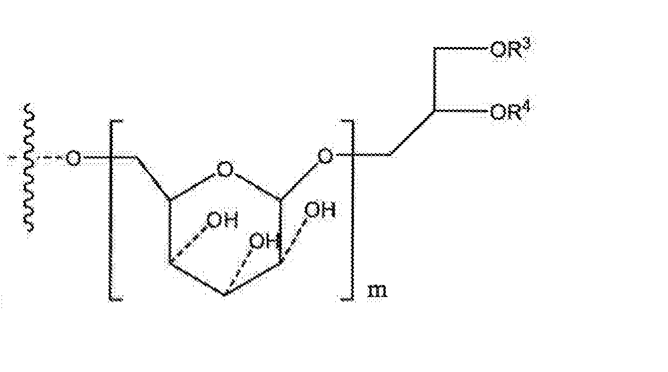 with R³ and R4 independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures;
preferably wherein the lipoteichoic acid is a structure according to general formula I 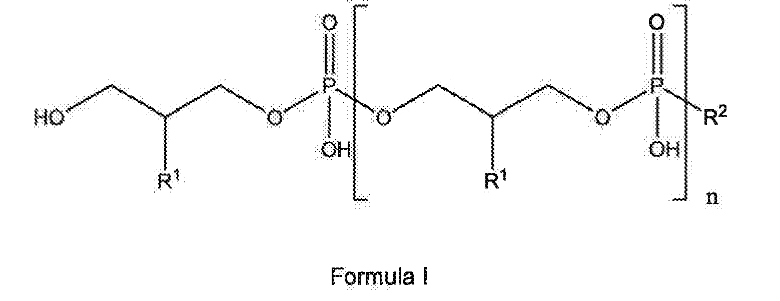 wherein
the mean value of n is 9,
R¹ is either OH or O-D-Alanyl and
R² is 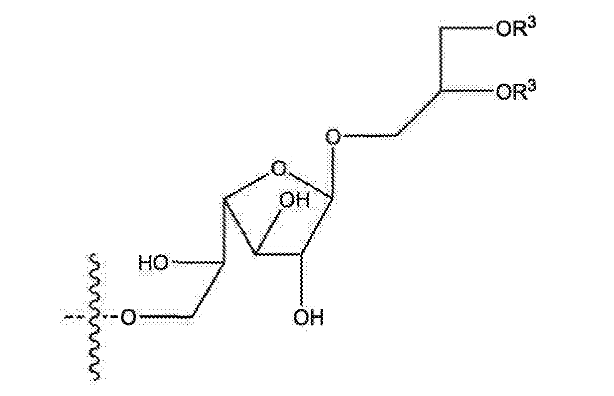 with R³ being residues of saturated or unsaturated fatty acids with 12, 14, 16 or 18 carbon atoms,
with the compounds of formula I being present either in free form or as physiologically acceptable salt; in form of any of their structural isomers including mixtures thereof, preferably in pure enantiomeric or diastereomeric form or any mixture thereof including racemic mixtures.

7. The pharmaceutical composition according to claim 2 wherein the lipoteichoic acid is a purified lipoteichoic acid isolated or isolatable from the Streptococcus sp. PT strain DSM 8747, preferably containing a beta-galactofuranosyl(1-3)glycerol-di-ester moiety.

8. The pharmaceutical composition according to claim 2 the lipoteichoic acid used according to the invention is a compound according to any of general formulas la, lb, lc, or ld 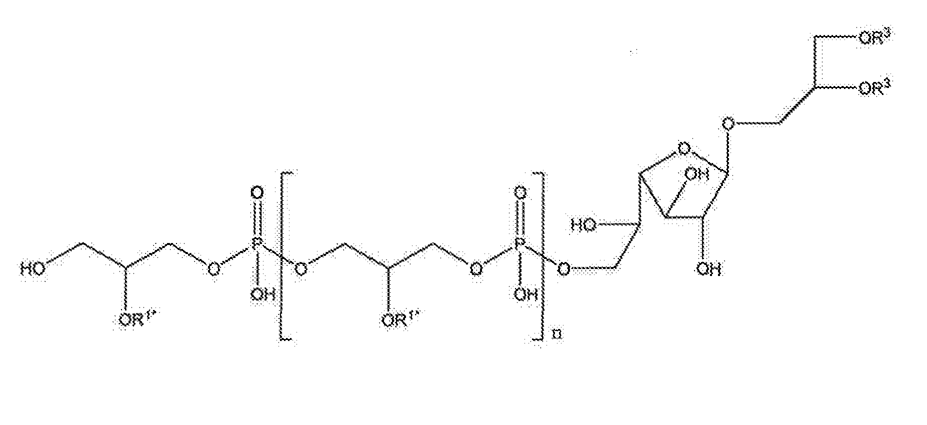 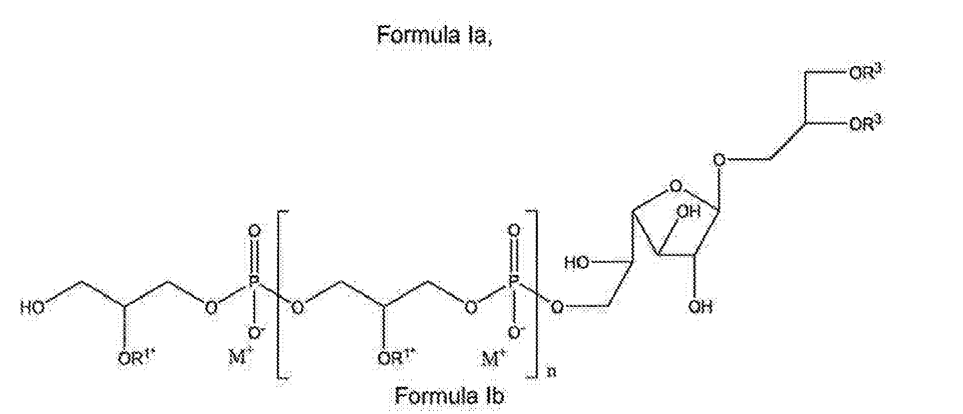 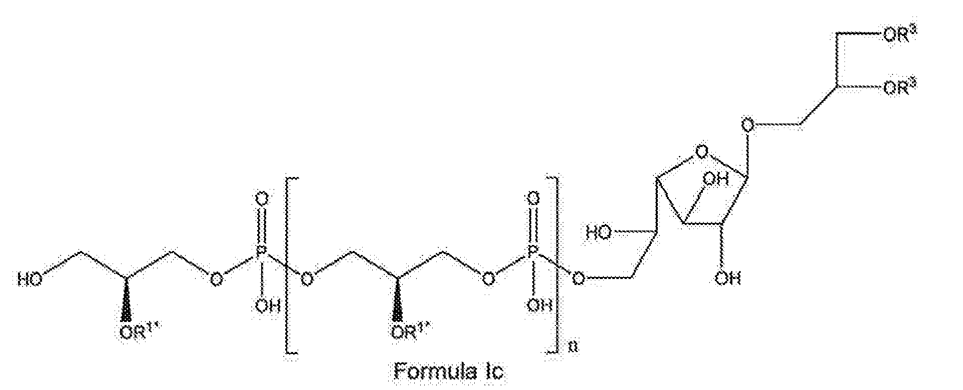 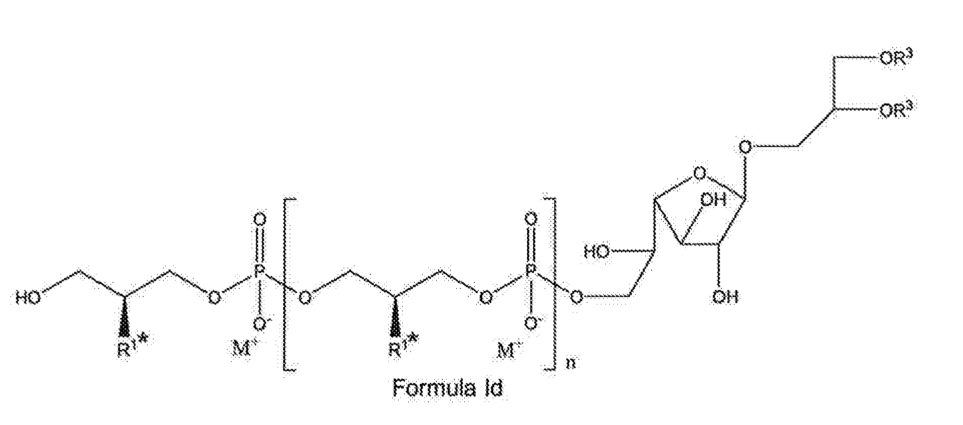 with
n having a mean value of 9, preferably being 6 to 12 while having a mean value of 9,
R^{1*} being either H or D-alanyl,
R³ being residues of saturated or unsaturated fatty acids with 12, 14, 16 or 18 carbon atoms, and
M⁺ being selected from positively charged ions, such as alkali metal ions or positively charged primary, secondary, tertiary or quaternary ammonium ions, preferably M⁺ being a sodium ion.

9. The pharmaceutical composition according to claim 2 wherein the lipoteichoic acid is LTA-T as a free acid or as a pharmaceutically acceptable salt or hydrate thereof, especially is LTA-T as a free acid or as a sodium salt.

10. The pharmaceutical composition according to any of claims 1 to 9 wherein the D-amino acid is selected from the group consisting of D-serine, D-alanine, D-lysine, D-arginine, D-glutamine D-asparagine or a pharmaceutically acceptable salt or hydrate thereof, preferably is D-alanine.

11. The pharmaceutical composition according to any of claims 1 to 10 wherein the D-amino acid is D-alanine and the teichoic acid is lipoteichoic acid, preferably wherein the D-amino acid is D-alanine and the teichoic acid is lipoteichoic acid T.

12. A pharmaceutical formulation comprising the pharmaceutical composition according to any of claims 1 to 11 and optionally one or more pharmaceutically acceptable excipients.

13. The pharmaceutical formulation according to claim 12 wherein the content of lipoteichoic acid T is 0.1 to 10 µmol, 0.5 to 2.0 µmol or 1 µmol and the content of D-alanine is 10 to 500 mg, preferably 40 to 250 mg or 50 mg; preferably the content of lipoteichoic acid T is 1 µmol and the content of D-alanine will be ca. 50 mg.

14. The pharmaceutical formulation according to any of claims 12 or 13 being a pharmaceutical dosage form prepared for injection.

15. The pharmaceutical composition according to any of claims 1 to 11 or the pharmaceutical formulation according to any of claims 12 to 15 for use in the treatment of cancer, tumors, malignant cells, elevated cholesterol levels in blood, pleural effusion or pneumothorax, preferably of cancer or pleural effusion; most preferably cancer or pleural effusion resulting from or combined with cancer.
